Europäisches Patentamt

European Patent Office

Office européen des brevets

(11) Veröffentlichungsnummer: **0 446 821 A2**

# (12) EUROPÄISCHE PATENTANMELDUNG

(21) Anmeldenummer: 91103640.8

(22) Anmeldetag: 09.03.91

(51) Int. Cl.5: **A61F 13/42**

(30) Priorität: **12.03.90 DE 4007813**

(43) Veröffentlichungstag der Anmeldung:
**18.09.91 Patentblatt 91/38**

(84) Benannte Vertragsstaaten:
**AT BE CH DE DK ES FR GB GR IT LI LU NL SE**

(71) Anmelder: **Kellerberg, Bernd**
**Oleanderstrasse 108**
**W-5010 Bergheim(DE)**

(72) Erfinder: **Kellerberg, Bernd**
**Oleanderstrasse 108**
**W-5010 Bergheim(DE)**

(74) Vertreter: **Langmaack, Jürgen, Dipl.-Ing. et al**
**Patentanwälte Maxton . Maxton . Langmaack**
**Goltsteinstrasse 93 Postfach 51 08 06**
**W-5000 Köln 51(DE)**

(54) **Gerät zur Prüfung des Durchfeuchtungsgrades von Windeln.**

(57) Bei modernen Windeln ist eine Durchfeuchtung durch Augenschein nicht festzustellen. Um eine Prüfung des Durchfeuchtungsgrades ohne Berührung mit der Hand zu ermöglichen, wird ein vorzugsweise stabförmiger Taster vorgeschlagen, der mit einem Fühlerkopf (3) versehen ist, der als Kondensatorplatte dient und über einen elektrischen Widerstand mit einem elektrischen Hochfrequenzgenerator elektrisch verbunden ist. Über eine damit elektrische Meß-schaltung (16) und eine Anzeige (7) kann nun der Durchfeuchtungsgrad durch einfaches Berühren der Windelaußenseite durch eine kapazitive Messung festgestellt werden.

Fig. 1

Die Erfindung betrifft ein Gerät zur Prüfung des Durchfeuchtungsgrades von Windeln, insbesondere feuchtigkeitsundurchlässigen Windelhosen mit einem Fühlerkopf, der mit einer elektrischen Stromversorgung und einer Meßschaltung mit Anzeigeeinrichtung verbunden ist.

In Krankenhäusern und Pflegeheimen besteht insbesondere bei Patienten mit Inkontinenz die Notwendigkeit, die Vorlagen bzw. Windeln daraufhin zu überprüfen, ob ein Wechsel, insbesondere ein vorzeitiger Wechsel, notwendig ist. Eine Prüfung durch Augenschein ist bei modernen Windeln und Vorlagen, die mit einer wasserdichten Folie versehen sind und in Form von geschlossenen Windelhosen angelegt werden, nicht mehr möglich, so daß hier das Pflegepersonal die Prüfung mit der Hand vornehmen muß, was schon aus hygienischen Gründen nicht zweckmäßig ist.

Aus der DE-OS 38 23 859 ist ein Gerät der eingangs bezeichneten Art bekannt, bei dem ein Kontaktkopf mit zwei mit Abstand zueinander angeordneten Elektroden versehen ist. Die Elektroden sind über eine Meßschaltung zur Messung der Veränderung des elektrischen (ohmschen) Widerstandes an eine elektrische Stromversorgung angeschlossen. Die Meßschaltung stellt einen offenen Meßkreis dar, der nur über die Elektroden geschlossen werden kann. Das bedeutet, daß der Taster am Patienten zwischen Körper und Windel eingeschoben werden muß. Das Einschieben muß hierbei so erfolgen, daß die Elektroden an der inneren Oberfläche der Windel zur Anlage kommen können. Die Toleranzgrenze der Meßschaltung ist hierbei so eingestellt, daß geringfügig durchfeuchtete Windeln, beispielsweise durch Schweißabsonderungen, noch keine Anzeige bewirken, da wegen des hohen Widerstandes in der Windeloberfläche nur ein geringer Strom fließt. Ist jedoch die Windel durchnäßt, so ergibt sich wegen der guten Leitfähigkeit des menschlichen Urins ein entsprechend geringer Widerstand und damit ein hoher Stromfluß, so daß über die Anzeige abgelesen werden kann, daß die Windel durchfeuchtet ist und gewechselt werden muß. Dieser vorbekannte Taster hat zwar den Vorteil, daß dem Pflegepersonal die Kontrolle ermöglicht wird, ohne daß die Windel unmittelbar mit der Hand berührt werden muß. Der Nachteil besteht jedoch darin, daß der Kontaktkopf mit Urin, aber auch mit Kot in Berührung kommt, so daß insbesondere in Krankenhäusern der Kontaktkopf nach jeder Benutzung sterilisiert werden muß, um hier die Verbreitung von Krankheitskeimen zu verhindern. Ein weiterer Nachteil des vorbekannten Gerätes besteht in dem relativ hohen Stromverbrauch, so daß bei Batteriebetrieb die Batterien häufig gewechselt werden müssen, bzw. bei der Ausrüstung mit einem wieder aufladbaren Akku im Schwesternzimmer dann ein Ladegerät vorgesehen werden muß, so daß die Schwester darauf achten muß, nach einem Rundgang das Gerät jeweils wieder auf das Ladegerät aufzustecken.

Der Erfindung liegt die Aufgabe zugrunde, ein Gerät der eingangs bezeichneten Art zu schaffen, das eine Messung des Durchfeuchtungsgrades ermöglicht, ohne daß hierbei unmittelbarer Kontakt mit dem Körper bzw. mit den Körperausscheidungen benötigt wird.

Diese Aufgabe wird gemäß der Erfindung dadurch gelöst, daß der Fühlerkopf aus einer Metallplatte besteht, die in einen Isolierwerkstoff eingebettet ist und die in bezug auf die zu prüfende Windelhose eine Kondensatorplatte bildet und die über einen elektrischen Widerstand mit einem elektrischen Hochfrequenzgenerator elektrisch verbunden ist, und daß dem Widerstand ein Spannungsmesser zum Messen des Spannungsabfalles über den Widerstand zugeordnet ist, dessen Signalausgang mit einer Signalschaltung zur Erzeugung des Anzeigesignals verbunden ist. Diese Anordnung hat den Vorteil, daß der Fühlerkopf von außen beispielsweise an die Folie der Windel angelegt werden kann. Die mit einer isolierenden Kunststoffumhüllung versehene Metallplatte auf der einen Seite und die mit einer entsprechenden saugfähigen Füllung, beispielsweise einem Granulat, versehenen Windel auf der anderen Seite bildet bei Anlage des Fühlerkopfes an der Windel praktisch einen Kondensator. Liegt nun an der Metallplatte eine hochfrequente elektrische Wechselspannung an, so ergibt sich in dem Augenblick eine Belastung des um den Fühlerkopf aufgebauten elektrostatischen Wechselfeldes, wenn eine Annäherung bzw. Berührung der Windelhose erfolgt, deren elektrisch neutrale, saugfähige Füllung eine ionisierbare Flüssigkeit enthält. Bei entsprechender Einjustierung der "Null-Lage" der Schaltungsanordnung bei trockener Windel ergibt sich im Falle der Anwesenheit von Flüssigkeit in der Windel aufgrund der Ladungsverschiebung ein geringer Stromfluß im Gerät selbst mit entsprechendem Spannungsabfall über den Widerstand. Dieser Spannungsabfall kann nun über den Spannungsmesser gemessen werden, wobei das gewonnene Meßsignal zur Erzeugung eines Anzeigesignals verwendet werden kann. Da der Fühlerkopf nur von außen an die Windel angelegt wird, kommt dieser bei den heute üblichen Windelhosen nicht mit den Ausscheidungen in Berührung, so daß hier nur die üblichen Desinfektionsmaßnahmen für Stationsgeräte vorzusehen sind. Ein weiterer Vorteil des erfindungsgemäßen Gerätes besteht darin, daß es sich um eine statische Messung handelt, da kein Stromfluß beim Meßvorgang am Fühlerkopf erfolgt. Damit ist bei einer Stromversorgung durch Batterien oder Akkus eine erheblich größere Betriebsdauer gewährleistet. Dies ermöglicht es einer Schwester, das Gerät ständig in der Tasche bei sich zu führen, wobei durch übliche Batterie-Checkanzeigen die Kontrolle über den Ladezustand der Batterie möglich ist. Der Begriff "elektrischer Widerstand" umfaßt nicht nur einen einfachen ohmschen Widerstand, sondern auch sogenannte Widerstandsschaltungen. Da durch die "Ladungsverschiebung" im Bereich der Flüssigkeit in der Windelhose nur ganz geringe Ströme fließen, muß der elektrische Widerstand schon im Hinblick auf den für die Spannungsmesser in solchen Fällen erforderlichen Operationsverstärker ausgelegt werden. Die Widerstandsschaltung ist hierbei als vorgespannter Arbeitspunkt entsprechend der Kennlinie des Operationsverstärkers ausgelegt. Derartige Widerstandsschaltungen sind dem Fachmann grundsätzlich geläufig, wenn er ganz geringe Ströme in üblichen Vorverstärkern etwa 10.000-fach verstärken muß.

In bevorzugter Ausgestaltung der Erfindung ist hierbei vorgesehen, daß der elektrische Hochfrequenzgenerator eine Wechselspannung zwischen 20 und 50 kHz erzeugt. Bei einer derartigen Arbeitsfrequenz lassen sich in dem hier in Betracht kommenden Bereich der Messung der Durchfeuchtung zuverlässige Anzeigesignale erzeugen, wobei für die Stromversorgung eine Spannung von 5 Volt ausreicht.

In weiterer Ausgestaltung der Erfindung ist vorgesehen, daß der Spannungsmesser durch einen Operationsverstärker gebildet wird, der eingangsseitig mit dem Ausgang des elektrischen Widerstands und ausgangsseitig mit dem Eingang eines Komparators verbunden ist, dessen zweiter Eingang mit dem Eingang des elektrischen Widerstands verbunden ist. Diese Anordnung erlaubt es, mit konventionellen elektrischen Bausteinen, den Stromfluß zwischen dem Eingang und dem Ausgang des elektrischen Widerstandes als Spannungsdifferenz zu erfassen, wenn bei der Anlage des Fühlerkopfes an der Außenseite einer feuchtigkeitsundurchlässigen Windel die Windelfüllung mit Feuchtigkeit durchsetzt ist. Die Meßeinrichtung kann hierbei so empfindlich ausgebildet werden, daß auch dann noch ein Signal auftritt, wenn eine mit Granulat gefüllte Windel, in der das Wasser chemisch gebunden wird, in einem Maße durchfeuchtet wird, daß bei einer Sicht- oder Fühlprüfung die Windel noch als trocken beurteilt würde. Das chemisch gebundene Wasser führt gleichwohl zu einer Auslösung eines Signals.

In einer zweckmäßigen Ausgestaltung der Erfindung ist vorgesehen, daß das Gerät mit einer akustischen und/oder optischen Anzeige versehen ist. Insbesondere eine optische Anzeige ist für die Kontrolle von inkontinenten Personen während der Nacht vorteilhaft, da bei einer Überprüfung weder die betreffende Person noch andere, im gleichen Zimmer schlafende Personen gestört werden.

Zweckmäßig ist es hierbei, wenn ein Wählschalter für die Auswahl der optischen und/oder akustischen Anzeige vorgesehen ist.

In weiterer vorteilhafter Ausgestaltung der Erfindung ist vorgesehen, daß der Meßschaltung eine Stromversorgungsschaltung vorgeschaltet ist, die zumindest eine Timerschaltung aufweist. Diese Anordnung hat den Vorteil, daß über die sogenannte Timerschaltung das Gerät selbsttätig auch dann abschaltet, wenn nach der Benutzung vergessen wird, den mechanischen Ein/Ausschalter zu betätigen. Derartige Timerschaltungen sind grundsätzlich für batteriebetriebene bzw. akkubetriebene Meßgeräte bekannt.

In besonders vorteilhafter Ausgestaltung der Erfindung ist ferner vorgesehen, daß die Stromversorgungsschaltung eine Spannungskontrollschaltung aufweist. Die Spannungskontrollschaltung ist hierbei so ausgelegt, daß bei einer Versorgungsspannung von 9 Volt mit Hilfe üblicher Blockbatterien oder Akkus für die Meßschaltung und den Spannungsmesser eine Betriebsspannung von 5 Volt zur Verfügung gestellt wird. Sinkt die Versorgungsspannung von 9 Volt auf eine Spannung von 5 Volt oder darunter ab, so wird ein optisches und/oder akustisches Signal ausgelöst, beispielsweise in der Form, daß bei einem Meßvorgang die Meßanzeige blinkt bzw. kein Dauerton sondern nur noch ein mehrfach unterbrochener Ton als Anzeige erscheint, so daß der Benutzer durch diese Intervallanzeige darauf hingewiesen wird, daß das Meßsignal nicht mehr zuverlässig ist und die Batterie ausgewechselt werden muß. Die Spannungsversorgungsschaltung in dieser Funktion für sich ist bekannt.

Während es grundsätzlich möglich ist, den Fühlerkopf mit einem Griff zu versehen und dann über ein Kabel mit einem in der Tasche befindlichen Schaltungskasten zu verbinden, ist in einer besonders vorteilhaften Ausgestaltung der Erfindung vorgesehen, daß der Fühlerkopf am freien Ende eines Taststabes angeordnet ist, in dem der elektrische Leiter verläuft und der mit einem gehäuseartigen Handgriff versehen ist, in dem die Meßschaltung einschließlich der Stromversorgungsschaltung und der Batterie angeordnet ist, und daß der Handgriff die Anzeigeeinrichtung trägt. Ein derartiges Gerät kann auch klein und leicht gebaut werden, so daß es von einer Krankenschwester bequem in der Kitteltasche mitge. führt werden kann. Zweckmäßig ist es hierbei, wenn der Taststab aus einem elastisch biegsamen Material hergestellt ist.

In einer vorteilhaften Ausgestaltung der Erfindung ist vorgesehen, daß der Ein/Ausschalter, der Stromversorgungsschalter und der Wählschalter für die Anzeige als gekapselte Schaltelemente ausgebildet sind. Dies ermöglicht es, den gehäuseartigen Handgriff bis auf das Batteriefach dicht verschlossen auszubilden, so daß bei einer Behandlung mit einer Desinfektionslösung keine Flüssigkeit in die elektrischen Teile im Gehäuseinnern eindringen kann. Derartige Schalter sind üblicherweise so ausgebildet, daß auf der Außenseite ein mechanisches Schieberelement angeordnet ist, das über entsprechende Magnete auf die im Gehäuseinnern angeordneten Schaltelemente berührungslos einwirkt. Sogenannte Folienschalter, wie sie für die Betätigung von Steuerungen im Naßbereich üblich sind, können auch hier eingesetzt werden.

In Ausgestaltung der Erfindung ist ferner vorgesehen, daß als Stromversorgung ein im gehäuseartigen Handgriff fest angeordneter, aufladbarer Akku vorgesehen ist, und daß nur die Ladekontakte nach außen geführt sind.

In einer anderen Ausgestaltung der Erfindung ist vorgesehen, daß der Fühlerkopf über ein Kabel mit einem Gehäuse verbunden ist, in dem die Meßschaltung einschließlich der Stromversorgung angeordnet ist und das mit einer Anzeigeeinrichtung verbunden ist. Eine derartige Ausbildung ist insbesondere für Rollstuhlfahrer oder Personen, die aufgrund ihrer Behinderung an einen Sessel gebunden sind, von Vorteil. Diese Ausbildung erlaubt es, den Fühlerkopf über entsprechende Befestigungsmittel in Form von Klemmen, Klettverschlußstreifen, Sicherheitsnadeln oder dergl. an der Unterkleidung zu befestigen und das über ein Kabel mit dem Fühlerkopf verbundene Gehäuse in einer Tasche der Oberbekleidung oder am Rollstuhl anzuordnen, so daß das Signal gut hör- und/oder sichtbar ist. Auch hier erweist sich der Vorteil des erfindungsgemäßen Gerätes dadurch, daß der Fühlerkopf klein, glattflächig und mit körpergerecht abgerundeten Kanten ausgebildet werden kann. Es ergeben sich keine Behinderungen oder Beeinträchtigungen, zumal der Fühlerkopf im Bereich von Körperpartien an der Unterkleidung befestigt werden kann, die nicht in einer vom Körpergewicht belasteten Zone liegen, gleichwohl ein lässiges "Erkennen" der Durchfeuchtung der Windel bei Inkontinenz gewährleisten. Die Anzeigeeinrichtung kann ebenfalls über ein Kabel vom Gehäuse getrennt als gesondertes Bauteil angeschlossen sein.

Die Erfindung wird anhand schematischer Zeichnungen für ein Ausführungsbeispiel näher dargestellt. Es zeigen:

Fig. 1    ein Handgerät in Vorderansicht,
Fig. 2    das Handgerät in Seitenansicht,
Fig. 3    eine Stirnansicht in Richtung des Pfeiles I in Fig. 1,
Fig. 4    ein Blockschaltbild,
Fig. 5    eine Anordnung für einen "stationären" Einsatz,
Fig. 6    eine Ausführungsform mit integriertem Ladeteil.

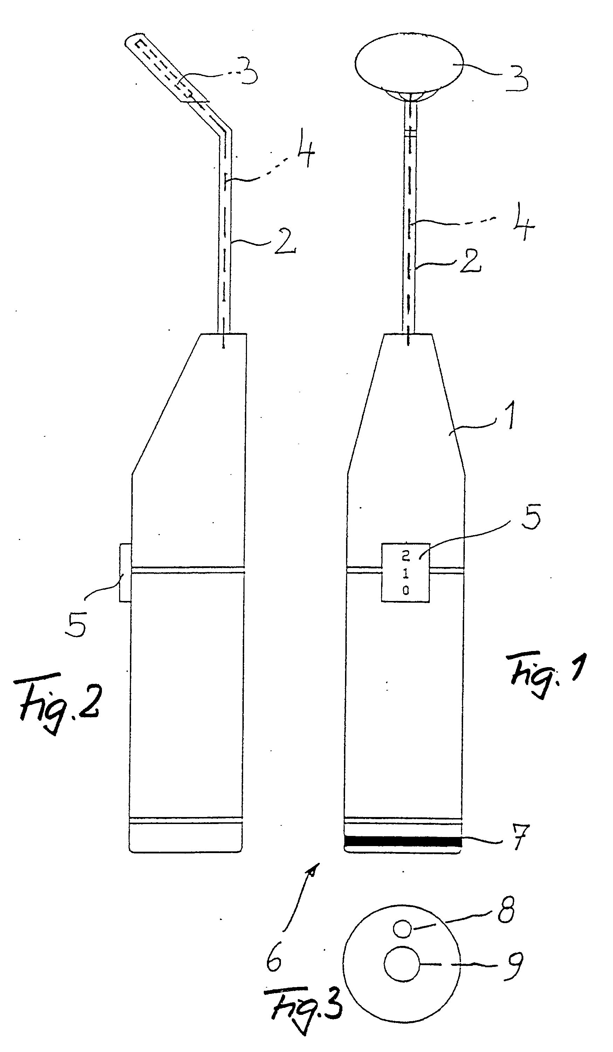

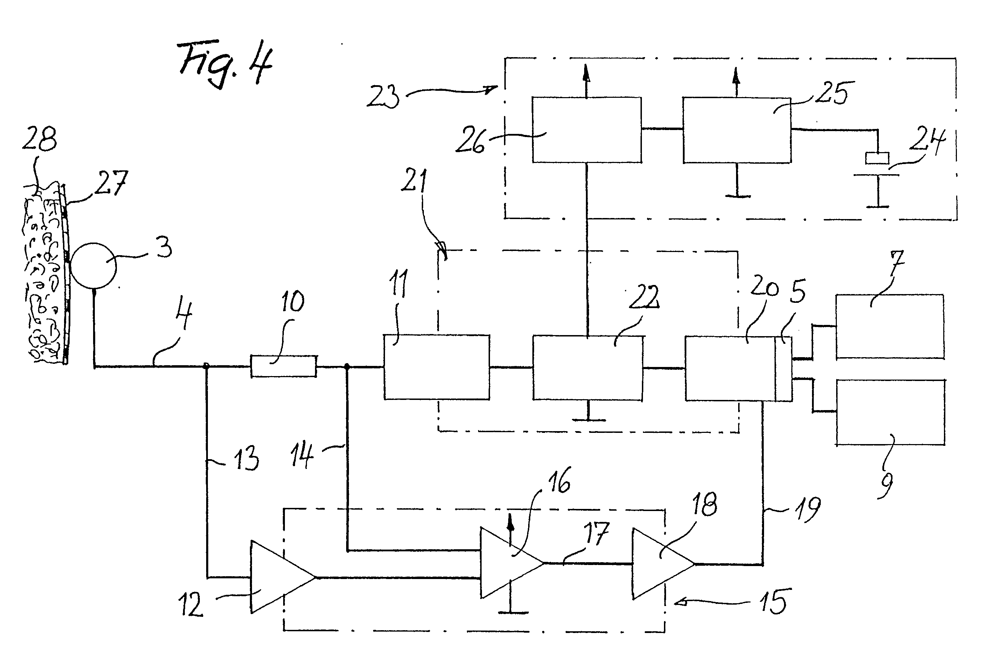

Das in Fig. 1 dargestellte Handgerät besteht aus einem gehäuseförmigen Handgriff 1, in dem die anhand von Fig. 4 näher erläuterte Schaltungsanordnung untergebracht ist. Am Handgriff 1 ist ein Taststab 2 befestigt, an dessen freiem Ende ein Fühlerkopf 3 in Form einer in einen elektrisch isolierenden Kunststoff eingebetteten Metallplatte befestigt ist. Der Taststab 2 ist, wie aus Fig. 2 ersichtlich, am freien Ende abgewinkelt, so daß er im Stehen vom Pflegepersonal bei einer im Bett liegenden Person unter den mit der Windelhose bedeckten Körperteil geschoben werden kann. Die Metallplatte 3 ist über einen im Taststab 2 verlaufenden elektrischen Leiter 4 mit der im Handgriff angeordneten Schaltung verbunden.

Auf der Außenseite des Handgriffs 1 ist ein Betätigungselement für ein im Gehäuseinnern angeordnetes elektrisches Schaltelement 5 angeordnet, mit dem das Gerät eingeschaltet und wieder ausgeschaltet werden kann. Bei dem dargestellten Ausführungsbeispiel ist der Schalter zugleich als Wählschalter ausgebildet, so daß die unterschiedlichen Anzeigearten optisch und/oder akustisch wahlweise vorgegeben werden können.

Am freien Ende 6 des Handgriffs 1 ist ferner die Anzeigeeinrichtung untergebracht. Diese besteht aus einem, zweckmäßigerweise an der freien Stirnseite des Handgriffs angebrachten piezo-akustischen Summers 7 und zwei vorzugsweise verschiedenfarbige Leuchtdioden 8 und 9. Die Leuchtdiode 8 kann beispielsweise weiß oder grün leuchten und zeigt lediglich an, daß das Gerät betriebsbereit ist, d.h. also, eingeschaltet ist. Die Leuchtdiode 9 leuchtet zweckmäßigerweise rot und leuchtet nur dann auf bei betriebsbereitem Gerät und wenn die Metallplatte 3 an eine Windel angelegt ist, die benäßt ist.

Das Blockschaltbild in Fig. 4 zeigt die wesentlichen Elemente der Schaltung in Form eines Blockschaltbildes. Die Metallplatte 3 ist hierbei mittels der Zuleitung 4 über eine elektrische Widerstandsanordnung 10 mit einem Hochfrequenzgenerator 11 verbunden. Die elektrische Widerstandsanordnung ist hierbei zwar als ohmscher Widerstand dargestellt, besteht jedoch aus einer Schaltungsanordnung, die auf einen aus der Kennlinie eines nachstehend noch näher erläuterten Operationsverstärkers 12 abgestimmt ist.

An die Zuleitung 4 ist, bezogen auf den Frequenzgenerator 11, vor und hinter dem Widerstandselement 10 je ein Abgriff 13 und 14 vorgesehen, der auf eine Spannungsmesserschaltung 15 aufgeschaltet ist. Dieser Spannungsmesserschaltung besteht im wesentlichen aus einem Komparator 16, auf den die Zuleitung 14 direkt aufgeschaltet ist. Die Zuleitung 13 ist über den vorstehend bereits erwähnten Operationsverstärker 12 auf den Komparator 16 aufgeschaltet, wobei dieser Operationsverstärker eine Signalverstärkung um etwa das 10.000-fache in der Form bewirkt, daß proportional zu dem in der Zuleitung 13 fließenden Strom am Ausgang eine Wechselspannung als Signal abgegriffen werden kann. Diese wird unter Gleichrichtung auf den Komparator 16 aufgeschaltet, der mit der halben Spannung vorgespannt ist. Ist bei Stromfluß über dem Widerstand 10 die vom Operationsverstärker 12 abgegebene Spannung größer als die Vorspannung am Komparator 16, dann wird ein Signal abgegeben. Der Singalausgang 17 des Komparators 16 ist wiederum auf einen Operationsverstärker 18 aufgeschaltet, dessen Ausgang 19 auf ein Logikteil 20 aufgeschaltet ist, dem der Wählschalter 5 zugeordnet ist. Die aus dem Frequenzgenerator 11 und dem Logikteil 20 im wesentlichen bestehende Signalschaltung 21 ist ferner noch mit einem Tongenerator 22 verschaltet, der mit dem Logikteil in Verbindung steht und der bei eingeschalteter Stromversorgung ständig eine Wechselspannung von etwa 1 bis 2 kHz erzeugt. Über den Wählschalter 5 kann nun entweder die optische Anzeige 9 eingeschaltet werden und je nach Zahl der Schaltstufen entweder nur auf den akustischen Signalgeber 7 oder zusätzlich zur optischen Anzeige 9 den akustischen Signalgeber 7 zuschalten.

Die Stromversorgung der Signalschaltung 21 und der Spannungsmesserschaltung 15 erfolgt über eine Versorgungsschaltung 23, die bei dem dargestellten Beispiel aus einer 9 Volt-Batterie 24 besteht, die mit ihrem Pluspol auf eine Timerschaltung 25 aufgeschaltet ist. Die Timerschaltung 25 ist hierbei so ausgelegt, daß nach einer vorgegebenen Betriebszeit von beispielsweise einer Minute das Gerät auch dann abschaltet, wenn der Wählschalter 5 noch auf "ein" geschaltet ist. Der Timerschaltung 25 ist eine Spannungskontrollschaltung 26 nachgeordnet, die so ausgelegt ist, daß die Signalschaltung 21 und die Spannungsmesserschaltung 15 mit einer Spannung von nur 5 Volt versorgt werden. Die Schaltung ist hierbei ferner so ausgebildet, daß bei einem Unterschreiten der Versorgungsspannung von 5 Volt bei Benutzung das optische und/oder das akustische Signal nur noch in Intervallen erfolgt, so daß hier für den Benutzer ein deutlicher Hinweis gegeben ist, daß die Batterie ausgewechselt werden muß. Anstelle einer Batterie kann auch ein aufladbarer Akku vorgesehen werden, wobei der Akku fest im Handgriff 1 installiert sein kann, während die Kontakte zur Verbindung mit dem Ladegerät nach außen geführt sind.

Wird das Gerät eingeschaltet, dann steht an der Metallplatte 3 eine Wechselspannung je nach Wahl des Hochfrequenzgenerators zwischen 20 und 50 kHz an, ohne daß ein Strom fließt. Wird nun die Metallplatte 3 an die Schutzfolie 27 einer Windelhose angelegt und ist die in der Windelhose enthaltene Füllung 28 durchnäßt, dann erfolgt durch die Flüssigkeit innerhalb der Windelfüllung 28 eine Belastung des elektrostatischen Wechselfeldes um die Metallplatte 3, so daß sich über den elektrischen Widerstand 10 ein wenn auch geringer Stromfluß ergibt, der dann nach entsprechender Verstärkung im Operationsverstärker 12 als Spannungsdifferenz im Komparator erfaßbar ist. Der Komparator kann nun so eingestellt werden, daß erst bei einer Mindestspannungsdifferenz ein Signal an das Logikteil 20 der Signalschaltung 21 abgegeben wird.

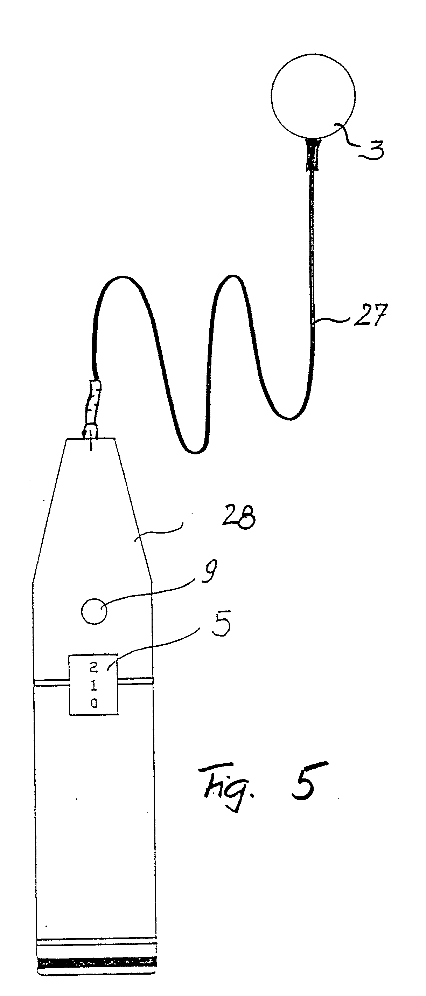

Wie Fig. 5 zeigt, kann der Fühlerkopf 3 anstelle des Taststabes auch über ein Kabel 27 mit einem Gehäuse 28 verbunden sein, so daß er auch "stationär" eingesetzt werden kann, beispielsweise durch Befestigen an der Unterkleidung. Das Gehäuse 28 kann dann in einer Tasche der Oberbekleidung getragen oder beispielsweise am Rollstuhl befestigt werden.

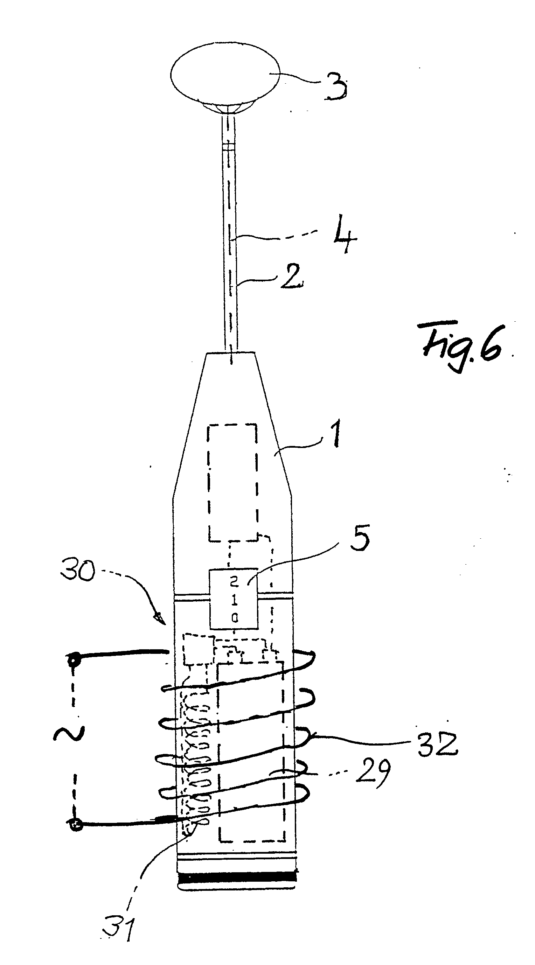

In Fig. 6 ist schematisch eine Ausführungsform dargestellt, die mit einem wiederaufladbaren Akku 29 und einer integrierten Ladeeinrichtung 30 versehen ist. Die Ladeeinrichtung 30 ist so ausgebildet, daß sie nur die Sekundärspule 31 mit der zugehörigen Gleichrichterschaltung enthält. Die mit der Stromversorgung verbundene Primärspule 32 ist hierbei als Tauchspule ausgebildet und in einem entsprechenden Gehäuse, das gleichzeitig als Halterung dient, an der Gebäudewand befestigt. Diese Anordnung ermöglicht es, das Gerät jeweils nach dem Gebrauch in die Halterung einzustecken, beispielsweise nach Beendigung einer Visite, so daß der Akku 29 wieder aufgeladen werden kann.

Vorzugsweise bei den stationär einzusetzenden Geräten ist es zweckmäßig, auch eine drahtlose Fernübertragung vorzusehen, beispielsweise bei der Verwendung in Alten- oder Pflegeheimen oder dergl., da es dann auch möglich ist, das Meßsignal zum Schwesternzimmer zu übertragen, um dort eine akustische oder optische Anzeige auszulösen. Durch die Auswahl unterschiedlicher Frequenzen ist es hierbei auch möglich, die Anzeige so zu bewerkstelligen, daß die diensttuende Schwester auch erkennen kann, welches Überwachungsgerät die Anzeige ausgelöst hat. Sie kann dann gezielt den betreffenden Patienten versorgen.

**Patentansprüche**

1. Gerät zum Prüfen des Durchfeuchtungsgrades von Windeln, insbesondere feuchtigkeitsundurchlässigen Windelhosen mit einem Fühlerkopf, der mit einer elektrischen Stromversorgung und einer Meßschaltung sowie einer Anzeigeeinrichtung verbunden ist, **dadurch gekennzeichnet,** daß der Fühlerkopf (3) aus einer Metallplatte besteht, die in einen Isolierwerkstoff eingebettet ist und die in bezug auf die zu prüfende Windelhose eine Kondensatorplatte bildet und die über einen elektrischen Widerstand (10) mit einem elektrischen Hochfrequenzgenerator (11) elektrisch verbunden ist und daß dem Widerstand (10) ein Spannungsmesser (15) zum Messen des Spannungsabfalles über den Widerstand (10) zugeordnet ist, dessen Signalausgang (19) mit einer Signalschaltung (21) zur Erzeugung eines Anzeigesignals verbunden ist.

2. Gerät nach Anspruch 1, dadurch gekennzeichnet, daß der elektrische Hochfrequenzgenerator (11) eine Wechselspannung zwischen 20 und 50 kHz erzeugt.

3. Gerät nach Anspruch 1 oder 2, dadurch gekennzeichnet, daß der Spannungsmesser (15) durch einen Operationsverstärker (12) gebildet wird, der eingangsseitig mit dem Ausgang des Widerstandes (10) und ausgangsseitig mit einem Eingang eines Komparators (16) verbunden ist, dessen zweiter Eingang mit dem Eingang des Widerstands (10) verbunden ist.

4. Gerät nach einem der Ansprüche 1 bis 3, dadurch gekennzeichnet, daß eine akustische und/oder optische Anzeige (7, 9) vorgesehen ist.

5. Gerät nach einem der Ansprüche 1 bis 4, dadurch gekennzeichnet, daß ein Wählschalter (5) für die Auswahl einer optischen und/oder akustischen Anzeige vorgesehen ist.

6. Gerät nach einem der Ansprüche 1 bis 5, dadurch gekennzeichnet, daß der Meßschaltung (15, 21) eine Stromversorgungsschaltung (23) vorgeschaltet ist, die zumindest eine Timerschaltung (25) aufweist.

7. Gerät nach Anspruch 6, dadurch gekennzeichnet, daß die Versorgungsschaltung (23) eine Spannungskontrollschaltung (26) aufweist.

8. Gerät nach einem der Ansprüche 1 bis 7, dadurch gekennzeichnet, daß der Fühlerkopf (3) am freien Ende eines Taststabes (2) angeordnet ist, in dem elektrische Leiter (4) verlaufen und der mit einem gehäuseartigen Handgriff (1) versehen ist, in dem die Meßschaltung (15, 21) einschließlich der Stromversorgung (23, 24) angeordnet ist und daß der Handgriff (1) die Anzeigeeinrichtung (7, 9) trägt.

9. Gerät nach einem der Ansprüche 1 bis 8, dadurch gekennzeichnet, daß der Taststab (2) aus einem elastisch biegsamen Material hergestellt ist.

10. Gerät nach einem der Ansprüche 1 bis 9, dadurch gekennzeichnet, daß der Ein/Ausschalter der Stromversorgungsschaltung (23) und/oder der Wählschalter (5) als gekapselte Schaltelemente ausgebildet sind.

11. Gerät nach einem der Ansprüche 1 bis 10, dadurch gekennzeichnet, daß der gehäuseartige Handgriff (1) bis auf ein Batteriefach hermetisch verschlossen ist.

12. Gerät nach einem der Ansprüche 1 bis 11, dadurch gekennzeichnet, daß die Stromversorgung (24) ein im gehäuseartigen Handgriff (1) fest angeordneter Akku ist und daß nur die Ladekontakte nach außen geführt sind.

13. Gerät nach einem der Ansprüche 1 bis 12, dadurch gekennzeichnet, daß der Fühlerkopf (3') über ein Kabel mit einem Gehäuse (1, 28) verbunden ist, in dem die Meßschaltung einschließlich der Stromversorgung angeordnet ist und das mit einer Anzeigeeinrichtung verbunden ist.

14. Gerät nach einem der Ansprüche 1 bis 13, dadurch gekennzeichnet, daß der Fühlerkopf (3') mit Befestigungsmitteln versehen ist.

15. Gerät nach einem der Ansprüche 1 bis 14, dadurch gekennzeichnet, daß der zur Stromversorgung dienende Akku (29) im Gehäuse (1, 28) fest angeordnet ist und daß der Ladegleichrichter mit der Sekundärspule (30) des Ladetrafos im Gehäuse fest angeordnet ist und über eine von außen an das Gehäuse anlegbare Primärspule eines Netzteiles der Ladespannung beaufschlagbar ist.

Fig. 2

Fig. 1

Fig. 3

Fig. 4

*Fig. 5*

Fig. 6